# EUROPEAN PATENT SPECIFICATION

(11) **EP 1 904 032 B1**
(45) Date of publication and mention of the grant of the patent: **08.12.2010**
(21) Application number: 06755740.5
(22) Date of filing: 07.07.2006
(51) Int. Cl.: A61K 9/12, A61K 31/4468

(54) **FENTANYL FORMULATION CONTAINING AN ESSENTIAL OIL**
FENTANYL-FORMULIERUNG MIT EINEM ESSENTIELLEN ÖL
FORMULATION DE FENTANYLE CONTENANT UNE HUILE ESSENTIELLE

(30) Priority: 08.07.2005 GB 0514043
(43) Date of publication of application: 02.04.2008
(73) Proprietor: Sosei R&D Ltd., Little Chesterford Saffron Walden Essex CB10 1XL (GB)
(72) Inventor: BOOLES, Clive Pharmasol Ltd North Way, Hampshire SP10 5AZ (GB); ROSS, Calvin Wellingham Farm, Dereham Norfolk NR19 2JF (GB)
(74) Representative: Gibson, Mark
(86) International application number: PCT/GB2006/002526
(87) International publication number: WO 2007/007059

(56) References cited:
- WO-A2-01/97780
- WO-A2-2004/080382

## Description

### Field of the Invention

This invention relates to a novel formulation of fentanyl containing an essential oil.

### Background of the Invention

Fentanyl is an analgesic. The drug is particularly useful when administered sublingually.

WO2004/080382 discloses a sprayable composition comprising fentanyl, e.g. as the free base, typically together with water, a polar solvent, citrate buffer and menthol. The given amount of menthol is 0.25% to 7.5%.

### Summary of the Invention

It has now been found that the menthol content in a fentanyl formulation is more important than was previously thought. If the amount is too high, and especially if it is more than 0.5%, crystals may form on scale-up.

According to the present invention, a formulation of the type described in WO2004/080382 is modified in that it contains menthol in an amount of less than 0.25%. Such a formulation has good long-term chemical and physical stability.

### Description of Preferred Embodiments

A formulation of the invention is preferably administered sublingually as a spray. It is well tolerated when administered to the sensitive sublingual mucosa, and the administration results in rapid onset of the therapeutic effect of the fentanyl.

A formulation of the present invention is preferably free of any propellant. In particular, by being water-based, it avoids the issues associated with using pressurised hydrofluorocarbon propellants. The formulation is preferably partially pressurised and free of propellants such as volatile chlorofluorocarbons (e.g. propellant 12), volatile hydrofluoroalkanes (e.g. 1, 1, 1, 2-tetrafluoroethane and 1, 1, 1, 2, 3, 3, 3-heptafluoro-n-propane), volatile alkanes (e.g. propane, butane) and other substances which have significant vapour pressure at ambient temperature and pressure.

In one embodiment of the present invention, the formulation is a solution, rather than a suspension. Whilst it is possible to spray a suspension, the fact that most suspensions settle means that the amount of active agent included in the dispensed dose may be variable. Although the effect of the settling of the suspension can be reduced to an extent by shaking the composition prior to spraying, some suspensions can settle very rapidly, so that there is still potential for variation of active agent content between doses.

Fentanyl may be employed in the form of a water-soluble physiologically acceptable salt, together with a polar organic solvent. Examples of suitable salts include hydrochloride, chloride, sulphate, tartrate and citrate. Preferably, fentanyl is employed as the free base.

Preferably, the fentanyl (which will herein be understood to include a physiologically acceptable salt thereof) is employed in the formulation at a concentration of 0.1 to 10 mg/ml, preferably 0.5 to 4.4 mg/ml (where weight is expressed as weight of fentanyl free base).

Examples of polar organic solvents that may be used to enhance the solubility of fentanyl in water include lower alcohols (e.g. C₂₋₄ alcohols) such as ethanol, lower polyols (e.g. C₂₋₄ polyols) such as glycerol and propylene glycol, and polyethylene glycols such as PEG200 and PEG400.

Mixtures of the above substances may be used. The preferred polar organic solvent is ethanol.

In another embodiment of the present invention, the formulation does not include ethanol. Indeed, the formulation may be substantially free of any alcohol, or completely free of alcohol. Where the composition is free of alcohol, the carrier used is preferably a polyol. The preferred polyols include propylene glycol and glycerol.

Generally speaking, it will be desired to employ the least amount of polar organic solvent necessary (or a modest excess over that necessary) to adequately solubilise the fentanyl, such that the fentanyl remains in solution under the conditions of likely usage or exposure. The concentration of polar organic solvent is preferably between 6 and 50%, more preferably 20-45%, especially 35-42%.

Preferably the water meets the USP (US Pharmacopoeia) or EP (European Pharmacopoeia) "Purified Water" standards.

The properties of the formulation may be improved by including one or more additional formulations components.

One such additional component is a buffer. The buffer is preferably adapted to stabilise the pH of the formulation at pH 7.4 to 8.5, preferably at pH 8.0 to 8.5, more preferably at 8.1 to 8.3, or around 8.2. At higher pH values, the bioavailability of the formulation may be improved relative to lower pH values (e.g. nearer pH 6). Examplary buffer systems include sodium acetate/acetic acid, ammonium acetate/disodium edetate, boric acid/sodium hydroxide, orthophosphoric acid/sodium hydroxide, sodium hydrogen carbonate/sodium carbonate, disodium hydrogen orthophosphate/citric acid (taken from the British Pharmacopoeia). The preference is use of a citrate buffer, e.g. a buffer comprising citric acid, sodium citrate and sodium hydroxide.

The concentration of the aqueous component (water or more preferably aqueous buffer) of the formulation of the present invention is preferably 50-94%, more preferably 55-80%, and especially 58-65%.

It may be desirable to include one or more of the following components in the formulation.
1) Sweeteners, flavouring or taste-masking agents (to improve patient acceptability), for example vanilla, pineapple extract, saccharin and sodium saccharin.
2) Moisturising agents (to improve patient comfort and overcome the drying tendency of ethanol and other polar organic solvents), for example pineapple extract, lanolin, polypropylene glycol, and polyethylene glycol.
3) Mucoadherents (in order to increase residency time on the mucosa), for example carboxyvinyl polymers, chitosans, polyacrylic acid, gelatin and polyvinyl pyrrolidone.
4) Preservatives (to improve long term resistance to microbial contamination), for example ethanol, sodium metabisulphite, benzalkonium chloride and Nipas.
5) Antioxidants, for example alkyl gallates, butylated hydroxyanisole, butylated hydroxytoluene, nordihydroguaiaretic acid, tocopherols, ascorbic acid and sodium metabisulphite.
6) Anionic surfactants, for example magnesium stearate, sodium cetostearyl sulphate, sodium lauryl sulphate, sulphated caster oil, sodium oleate, sodium stearyl fumarate and sodium tetradecyl sulphate.
7) Nonionic surfactants, for example glyceryl monostearate, macrogol cetostearyl ethers, poloxamers, polyoxyl stearates, polysorbates, sorbitan esters, sucrose esters, tyloxapol, propylene glycol monostearate, quillaia, polyoxyl caster oils, nonoxinols, lecithins and derivatives, oleic acid and derivatives, and oleyl alcohol and derivatives.
8) Foaming agents, for example alginic acid and salts, propylene glycol alginate, sodium lauryl sulphate, sodium cetostearyl sulphate, carbomers and hydroxyethylcellulose.

Some of the components proposed above may already be included in the composition of the present invention for other purposes. Suitable moisturising agents include, for example, the polar organic solvents such as glycols, especially propylene glycol, and the liquid polyethylene glycols, glycerol, methylcellulose, hypromellose, hydroxypropylcellulose, and many other substituted celluloses.

A versatile component, which improves the acceptability and other properties of the formulation, is menthol. Menthol, as well as flavouring the formulation, has moisturising effect. It may also have effect as a penetration enhancer. Menthol is employed in a concentration range of less than 0.25%, at least 0.01% or 0.1%, up to 0.2%, the optimum being 0.1%. Any increase in this percentage in scale-up batches may result in crystallisation and would be less suitable for commercial/clinical use.

One particular advantage of menthol is that it is compatible with fentanyl in a spray formulation. By contrast, peppermint oil (of which menthol is one component), may cause fentanyl to degrade.

In an embodiment of the invention, the formulation contains a sweetener. The preferred sweetener is saccharin or a physiologically acceptable salt thereof such as saccharin sodium. Preferably, the concentration of sodium saccharin or physiologically acceptable salt thereof is around 0.1-0.5%, e.g. around 0.28%.

Preferably the formulation contains saccharin. Surprisingly, we have found that the longer-term stability of formulations containing saccharin is better than the stability of those containing saccharin sodium.

It is not generally necessary to include a preservative in the formulation when ethanol is present, due to its preservative qualities.

Formulations of the invention are useful in analgesia and in the treatment of pain, e.g. moderate to severe pain. A therapeutically effective amount of the formulation should be used, and this can readily be determined by one of ordinary skill in the art.

Formulations according to the invention are preferably packaged as a bulk solution containing multiple doses in a pump spray system comprising a sealed container fitted with a metering pump. Such a container preferably contains between 20 to 200 doses. Example containers are those made out of plastics, glass and metal (e.g. aluminium); glass containers are preferred. Glass containers have the advantage that the contents of the container can be seen (i.e. it is possible to determine visually when the contents are about to run out). Furthermore, glass containers are less susceptible to tampering, which is an important consideration for narcotic substances.

Preferably, a glass container is coated on the exterior with a suitable moulded film of a plastics material, to protect against shattering. For example, the film may be of polypropylene. The material may be coloured and contain a UV absorber. Optionally, the interior of the containers can be coated to enhance stability of the product. Coatings include polymers and lacquers but also silicone dioxide can be used to line the inside of the container with an unreactive coating.

Another aspect of the invention is a metered dose dispensing system comprising a sealed container containing a formulation of the invention fitted with a metering pump, an actuator and a channelling device. The metered dose dispensing system is preferably adapted for sublingual administration.

Suitable metering pumps include those adapted for dispensation with the container in the upright or inverted orientation. Preferably the metering chamber is adapted for dispensation with the container in the upright orientation since this facilitates administration under the tongue. Accordingly, the metering chamber will be in communication with the bulk formulation by means of a dip-tube.

Examples of suitable metering pumps are those manufactured by Valois and illustrated in WO01/66089.

The metering pump is preferably a non-venting type with a dip tube. Such non-venting metering pumps may have, for example, a 100µl metering chamber capacity. Suitable materials for their construction include polypropylene and polyethylene. Suitable sealing materials, e.g. thermoplastic crimp gaskets suitable for the purpose, may be employed. In addition, a suitable aluminium ferrule purposely designed for crimping on to glass containers may be employed. Suitable grade stainless steel springs will preferably be adopted.

Preferably, the actuator is designed to deliver a sublingually effective dose. The package may be further enhanced by the fitting of a lock-out system to promote compliance by patients.

Typically a patient is treated by administration sublingually of 1 to 4 actuations, e.g. 1 or 2 actuations from the spray pump. It is an advantage of sublingual spray delivery that it is easy to titrate patients by 1 or 2 doses as required by a single actuation. This is not the case with other forms of drug delivery (patches, lozenges, tablets, suppositories).

The following Examples illustrate the invention, and also include comparative studies, providing evidence on which the present invention is based.

### Comparative Example 1

The following formulation was made up:

| | |
|---|---|
| Fentanyl Base | 0.43% |
| Absolute Ethanol | 44.69% |
| Menthol | 0.75% |
| Saccharin | 0.25% |
| Citrate Buffer | 53.88% |

The formulation from the bulk was filled into 10 ml glass Purguard containers and stored at 25°C. The units were inspected for consistency prior to stability testing. On inspection, the units had shown crystal growth at the base of the dip tubes. 10 units were further sonified, where the crystals dissolved but then returned.

The experiment was repeated, using three different batches of menthol which were dissolved in ethanol and subjected to citrate buffer solution. Three different levels of menthol, i.e. 0.25%, 0.50% and 0.75% were evaluated.

The units were made up in small quantities, implementing six Purguard bottles for each strength over the various percentages required. It was noted that it is possible to make up small amounts using 0.75% menthol, but on the scaling up of more than six units throughout the percentages, and even in some samples of 0.5%, crystals formed. The way to stop crystal growth was to use a lower strength of menthol.

The conclusion of the experiment indicated that menthol used in this particular type of pump spray formulation has to be carefully considered and in scale-up for commercial production, and that a level of about 0.1 % should be used. Crystallisation can be seen over time on storage at ambient temperatures in the glass containers, depending on the amount of menthol used. This is consistent between 200, 400 and 600 µg strengths.

### Example 2

An example of a formulation that illustrates the invention, and that is suitable for a sub-lingual pump spray, providing 20 µg per dose, comprises:

| | |
|---|---|
| Fentanyl Base | 0.2017% |
| Absolute Ethanol | 40.4371 % |
| Citrate Buffer | 59.0602% |
| Menthol | 0.1013% |
| Saccharin | 0.1997% |

Citrate buffer consists of:

| | |
|---|---|
| Citric acid anhydrous | 1.0% |
| Sodium citrate | 0.5% |
| Sodium hydroxide | 0.5% |
| Purified water | to 100% |

The buffer is adjusted to pH 8.2 with sodium hydroxide solution or citric acid solution as required before the buffer is finally made up to weight.

Large scale preparation of formulations containing 0.1% menthol has been successful. Batch sizes of 1 kg have been produced for each of various strengths. At this scale, approximately 2500 10 ml glass Purgard containers were filled with 4.3 g of solution.

## Claims

1. A pharmaceutical liquid spray formulation, comprising:
(a) fentanyl or a pharmaceutically acceptable salt thereof;
(b) water as carrier; and
(c) a polar organic solvent in sufficient amount to enhance the solubility of the fentanyl or salt thereof in the water; and
(d) menthol in an amount of less than 0.25% by weight.

2. A formulation according to claim 1, wherein the menthol is present in an amount of between 0.01% and 0.2%, such as between 0.1% and 0.2%, in particular 0.1%.

3. A formulation according to claim 1 or claim 2, wherein fentanyl is present as the free base.

4. A formulation according to any preceding claim, which is partially pressurised.

5. A formulation according to any preceding claim, wherein the fentanyl or salt is present at a concentration of 0.1 to 10 mg/ml.

6. A formulation according to any preceding claim, wherein the polar organic solvent is selected from ethanol, propylene glycol, glycerol, polyethylene glycol and mixtures thereof .

7. A formulation according to claim 6, wherein the polar organic solvent is ethanol.

8. A formulation according to any preceding claim, wherein the polar organic solvent is present in an amount of 6 to 50% w/w.

9. A formulation according to claim 8, wherein the polar organic solvent is present in an amount of 35 to 42% w/w.

10. A formulation according to any preceding claim, which is buffered.

11. A formulation according to claim 10, which is buffered with citrate buffer.

12. A formulation according to any preceding claim, whose pH is between 7.4 and 8.5.

13. A formulation according to claim 12, whose pH is 8.2.

14. A formulation according to any preceding claim, which contains a sweetener.

15. A formulation according to claim 14, wherein the sweetener is saccharin.

16. A formulation according to any preceding claim, for use in treating pain or as an analgesic.

17. A sealed container containing a plurality of doses of a formulation according to any of claims 1 to 16.

18. A container according to claim 17, which is made out of glass.

19. A metered dose dispensing system comprising a sealed container according to claim 17 or claim 18, and fitted with a metering pump, an actuator and a channelling device.

20. A dispensing system according to claim 19, containing a metering chamber which is adapted for dispensation with the container in the upright orientation and wherein the metering chamber is in communication with the formulation by means of a dip-tube.

## Patentansprüche

1. Pharmazeutische Flüssigkeitsspray-Formulierung, die Folgendes umfasst:
(a) Fentanyl oder ein pharmazeutisch akzeptables Salz davon;
(b) Wasser als Träger; und
(c) ein polares organisches Lösungsmittel in einer Menge, die ausreicht, um die Löslichkeit des Fentanyls oder Salzes davon in dem Wasser zu verbessern; und
(d) Menthol in einer Menge von weniger als 0.25 Gew.-%.

2. Formulierung nach Anspruch 1, wobei das Menthol in einer Menge zwischen 0.01% und 0.2%, z.B. zwischen 0.1% und 0.2%, inbesondere von 0.1 % vorliegt.

3. Formulierung nach Anspruch 1 oder Anspruch 2, wobei Fentanyl als die freie Base vorliegt.

4. Formulierung nach einem der vorherigen Ansprüche, die teilweise unter Druck steht.

5. Formulierung nach einem der vorherigen Ansprüche, wobei das Fentanyl oder Salz in einer Konzentration von 0.1 bis 10 mg/ml vorliegt.

6. Formulierung nach einem der vorherigen Ansprüche, wobei das polare organische Lösungsmittel ausgewählt ist aus Ethanol, Propylenglykol, Glycerol, Polyethylenglykol und Gemischen davon.

7. Formulierung nach Anspruch 6, wobei das polare organische Lösungsmittel Ethanol ist.

8. Formulierung nach einem der vorherigen Ansprüche, wobei das polare organische Lösungsmittel in einer Menge von 6 bis 50% w/w vorliegt.

9. Formulierung nach Anspruch 8, wobei das polare organische Lösungsmittel in einer Menge von 35 bis 42% w/w vorliegt.

10. Formulierung nach einem der vorherigen Ansprüche, die gepuffert ist.

11. Formulierung nach Anspruch 10, die mit Citratpuffer gepuffert ist.

12. Formulierung nach einem der vorherigen Ansprüche, deren pH-Wert zwischen 7.4 und 8.5 liegt.

13. Formulierung nach Anspruch 12, deren pH-Wert 8.2 ist.

14. Formulierung nach einem der vorherigen Ansprüche, die ein Süßungsmittel enthält.

15. Formulierung nach Anspruch 14, wobei das Süßungsmittel Saccharin ist.

16. Formulierung nach einem der vorherigen Ansprüche für die Verwendung bei der Behandlung von Schmerzen oder als Analgetikum.

17. Geschlossener Behälter, der mehrere Dosen einer Formulierung nach einem der Ansprüche 1 bis 16 enthält.

18. Behälter nach Anspruch 17, der aus Glas gefertigt ist.

19. System zum Spenden einer dosierten Menge, das einen geschlossenen Behälter nach Anspruch 17 oder Anspruch 18 umfasst und mit einer Dosierpumpe, einem Aktuator und einer Kanalisiervorrichtung ausgestattet ist.

20. Spendersystem nach Anspruch 19, das eine Dosierkammer enthält, die zum Spenden mit dem Behälter in der aufrechten Orientierung ausgelegt ist, und wobei die Dosierkammer über ein Tauchrohr mit der Formulierung in Verbindung ist.

## Revendications

1. Formulation pharmaceutique en aérosol liquide comprenant :
(a) le fentanyl ou un sel pharmaceutiquement acceptable de celui-ci ;
(b) l'eau comme véhicule ; et
(c) un solvant organique polaire en une quantité suffisante pour augmenter la solubilité du fentanyl ou du sel de celui-ci dans l'eau ; et
(d) le menthol en une quantité inférieure à 0.25 % en poids.

2. Formulation selon la revendication 1, où le menthol est présent en une quantité comprise entre 0.01 % et 0.2 %, par exemple entre 0.1 % et 0.2 %, en particulier à 0.1 %.

3. Formulation selon la revendication 1 ou la revendication 2, où le fentanyl est présent sous la forme de la base libre.

4. Formulation selon l'une quelconque des revendications précédentes, qui est partiellement pressurisée.

5. Formulation selon l'une quelconque des revendications précédentes, où le fentanyl ou son sel est présent à une concentration comprise entre 0.1 et 10 mg/ml.

6. Formulation selon l'une quelconque des revendications précédentes, où le solvant organique polaire est sélectionné parmi l'éthanol, le propylèneglycol, le glycérol, le polyéthylène-glycol et des mélanges de ceux-ci.

7. Formulation selon la revendication 6, où le solvant organique polaire est l'éthanol.

8. Formulation selon l'une quelconque des revendications précédentes, où le solvant organique polaire est présent en une quantité comprise entre 6 et 50 % p/p.

9. Formulation selon la revendication 8, où le solvant organique polaire est présent en une quantité comprise entre 35 et 42 % p/p.

10. Formulation selon l'une quelconque des revendications précédentes, qui est tamponnée.

11. Formulation selon la revendication 10, qui est tamponnée avec un tampon citrate.

12. Formulation selon l'une quelconque des revendications précédentes, dont le pH est compris entre 7.4 et 8.5.

13. Formulation selon la revendication 12, dont le pH est de 8.2.

14. Formulation selon l'une quelconque des revendications précédentes, qui contient un édulcorant.

15. Formulation selon la revendication 14, où l'édulcorant est la saccharine.

16. Formulation selon l'une quelconque des revendications précédentes, en vue d'une utilisation en traitement d'une douleur ou comme analgésique.

17. Récipient scellé contenant une pluralité de doses d'une formulation selon l'une quelconque des revendications 1 à 16.

18. Récipient selon la revendication 17, qui est fabriqué à partir de verre.

19. Système distributeur doseur comprenant un récipient scellé selon la revendication 17 ou la revendication 18 et équipé d'une pompe de dosage, d'un dispositif déclencheur et d'un dispositif d'acheminement.

20. Système distributeur selon la revendication 19 contenant une chambre de dosage qui est adaptée à la délivrance quand le récipient est orienté à la verticale et qui est en communication avec la formulation au moyen d'un tube plongeur.
